# EUROPEAN PATENT SPECIFICATION

(11) **EP 1 239 795 B1**
(45) Date of publication and mention of the grant of the patent: **06.12.2006**
(21) Application number: 00988113.7
(22) Date of filing: 15.12.2000
(51) Int. Cl.: A61F 2/24

(54) **ENHANCED VISUALIZATION OF MEDICAL IMPLANTS**
VERBESSERTE SICHTBARKEIT MEDIZINISCHER IMPLANTATE
VISUALISATION AMELIOREE DES IMPLANTS MEDICAUX

(30) Priority: 23.12.1999 US 471511
(43) Date of publication of application: 18.09.2002
(73) Proprietor: Edwards Lifesciences Corporation, Irvine, CA 92625 (US)
(72) Inventor: BESSLER, Anita, B., Laguna Hills, CA 92653 (US); HELMUS, Michael, N., Worcester, Massachusetts 01602 (US)
(74) Representative: Eke, Philippa Dianne
(86) International application number: PCT/US2000/034251
(87) International publication number: WO 2001/047438

(56) References cited:
- EP-A- 0 894 503
- EP-A- 0 916 317
- WO-A-97/24989
- WO-A-99/17680
- FR-A- 2 708 458

## Description

### Field of the Invention

The present invention relates to devices for improving the visualization of medical implants and, more typically, to implants that have structure incorporated therein to enhance their visibility to the naked eye.

### Background of the Invention

Various surgical procedures are currently performed to investigate, diagnose, and treat diseases of the heart and the great vessels of the thorax. Such procedures include repair and replacement of heart valves, repair of septal defects, treatment of aneurysms, and other procedures in which interventional devices are introduced into the interior of the heart or a great vessel. Heart valve surgery, in particular, is of particular relevance to the present invention. Various surgical techniques to repair or replace a diseased or damaged heart valve are known, including annuloplasty, resection, comissurotomy, shortening of the valve chordae, de-calcification of valves and annulus tissue, and in the so-called bow-tie technique, etc..

Ultimately, the valves leaflets may be excised and replaced with a mechanical or biological prosthesis. Such prostheses include pivoting disk-type mechanical valves, stented tissue valves, and so-called stentless homograft or allograft valves. In each of these, the conventional implantation technique is to suture the valve to the sculpted annulus from which the natural valve leaflets have been excised. Such procedures are well-known having been practiced since the 1960s.

Many current treatment techniques of the heart or great vessels of the thorax require a gross thoracotomy, usually consisting of a median sternotomy, to gain access to the thoracic cavity. A saw or other cutting instrument is used to cut the sternum longitudinally, allowing two opposed halves of the anterior or ventral portion of the rib cage to be spread apart. The surgical team may then directly visualize and operate upon the heart and other thoracic structures through the large opening in the thoracic cavity created. Unfortunately, such highly invasive procedures are extremely traumatic to the patient, present a substantial risk of complications, and result in a relatively long patient recovery time.

Less invasive surgical procedures have recently been developed which avoid the need for a gross thoracotomy. In such procedures, typically termed "minimally-invasive," access to the thoracic cavity is obtained through one or more relatively small access channels, typically formed through the intercostal spaces of the rib cage. Surgical instruments can then be inserted through the access channels to therapeutically treat the heart or other thoracic structures. Alternative minimally-invasive procedures involve a percutaneous approach.

As mentioned above, many cardiac surgical procedures require the implantation of a prosthesis using sutures. For example, a heart valve prostheses or annuloplasty ring is usually sutured to the annulus tissue. Placing sutures in the heart or other tissue that is accessed from outside of the patient's chest through small access channels present a variety of difficulties. Maneuverability is limited by the small opening and length of the access channel, and visibility is likewise impaired by the remote location of the surgery field and lack of ambient light. Moreover, correct placement and orientation of the particular implant is difficult in such minimally-invasive surgeries, even prior to the difficult task of suturing. Although minimally-invasive cardiac surgical techniques promised a significant reduction in patient trauma, risk, and recovery time, the difficulties imposed and corresponding training required has tended to limit growth in the number of surgeons performing such techniques. As a result, most surgeons continue to rely on the well-established, although imperfect, gross thoracotomy.

A variety of devices have been developed to facilitate minimally-invasive surgeries. Most of these devices focus on relatively complex mechanical structures for remotely or even robotically manipulating and installing implants. For example, U.S. patent No. 5,860,992 to Daniel, et al., discloses an endoscopic suturing device including curved suture needles that are hinged about the distal end and remotely actuated to pass through a sewing ring of a heart valve and adjacent tissue. The procedure is assisted through the use of an endoscope passed through the access port in parallel with the suturing device. This type of complex device is typical in the field as an answer to the drawbacks of minimally-invasive surgery. Unfortunately, such devices require extra training to operate and are sometimes not widely accepted for this reason.

Visibility of minimally-invasive procedures is accomplished through the use of endoscopes, or other such insertable magnification and/or illumination devices. Alternatively, many surgeries, especially graft implant procedures, utilize radiopaque markers on the implant and an external imager. Each of these techniques has drawbacks. For example, the image generated by an endoscope can be viewed through some type of ocular, or on a video display screen adjacent the surgery. Although great advances have been made in this respect, these systems are relatively expensive and all require sterilization or shielding in the operating room. Furthermore, even with a light source and endoscopic lens in the minimally-invasive surgical field, visibility of the implant and surrounding anatomical features remains limited. Systems for externally viewing radiopaque markers on implants are also expensive, and the images produced are not as clear as either direct visualization or endoscopically-assisted visualization.

EP-A-0 916 317 discloses a medical implant comprising a stent and a reflective structure on the stent, for example a gold or platinum coating.

Non-radiopaque markers on implants are also used to orient the implant with respect to a particular anatomical structure. For example, annuloplasty rings typically include a pair of commissure markers in a form of thread colored , differently than the rest of the ring, which is typically white. Likewise, vascular grafts may have orientation markers-incorporated therein. Stentless heart valves may have a thread pattern visible from the exterior of the tubular aortic wall to guide the surgeon in trimming the valve for partial root replacements. Although such non-radiopaque markers facilitate implantation of the prosthesis, they are still difficult to see in a minimally-invasive surgeries.

Despite much development in the minimally-invasive surgical field, there remains a need for improved visibility of medical implants.

### Summary of the Invention

The present invention provides apparatus to enhance the visibility of medical implants, surgical sites, or tools used for the implant procedure. The invention is especially advantageous for minimally-invasive surgeries in which full illumination of the surgical site may be hindered by the necessity of a deep access passage. The enhanced visibility structure on the implant, surgical site, or tool, can be provided in a number of ways, generally defined as luminescent.

The present invention provides the medical implant of independent claim 1. The dependent claims specify preferred but optional features.

In one aspect, the present invention provides a medical implant having structure with enhanced visibility thereon to facilitate location of the structure in low light environments. The enhanced visibility structure is luminescent; that is the structure may be fluorescent, phosphorescent, or chemiluminescent.

In another aspect, the present invention provides a cardiovascular implant system including attachment structure having enhanced visibility. The attachment structure is luminescent. The attachment structure may comprise sutures, wherein the cardiovascular implant includes a suture-permeable portion. Furthermore, enhanced visibility markings on the suture-permeable portion may be provided. In another aspect, the system may include an installation tool having an enhanced visibility marker thereon. Finally, the system may include the implant, the attachment structure, and an installation tool, all which have enhanced visibility markers thereon.

The invention also provides a cardiovascular implant having enhanced visibility including a fabric portion and an enhanced-visibility marking thereon. The implant may be a heart valve, and the fabric portion is a sewing ring. The enhanced-visibility marking may be provided on the entire sewing ring, on only a portion for rotational orientation, or along lines delineating either an inner or outer boundary thereof. Alternatively, the enhanced-visibility marking may indicate upstanding commissures of the heart valve. The implant may be an annuloplasty ring, with the enhanced-visibility marking on the entire ring, or on portions thereof

A method of installing a medical implant in the body comprises preparing an implantation site within the body including marking at least one anatomical landmark at the implantation site with structure having enhanced visibility. The method further includes installing the medical implant at the implantation site with the help of the enhanced visibility structure. The structure may comprise a suture or a biocompatible compound. The anatomical landmark may be the annulus of a heart valve or the commissures thereof. The method preferably includes providing structure on the medical implant having enhanced visibility, and registering that structure with the enhanced-visibility structure on the anatomical landmark.

A further understanding of the nature advantages of the invention will become apparent by reference to the remaining portions of the specification and drawings.

### Brief Description of the Drawings

Figure 1 is a partially cutaway perspective view of an exemplary heart valve of the present invention having various enhanced visibility structure thereon;
Figure 2 is a radial sectional view through one edge of the heart valve of Figure 1 attached to a heart valve annulus;
Figure 3 is an elevational view of an alternative heart valve of the present invention having enhanced visibility structure thereon;
Figure 4 is an elevational view of an annulus sizer of the present invention having enhanced visibility structure thereon;
Figure 5 is an exploded perspective view of an exemplary flexible heart valve and corresponding holder of the present invention having enhanced visibility structure thereon;
Figures 6A-6C illustrate a number of steps in the implantation of a stentless heart valve of the present invention utilizing enhanced visibility structure in accordance with the present invention;
Figure 7 is a perspective view of a step in the implantation of a continuous annuloplasty ring of the present invention having enhanced visibility structure thereon;
Figure 8 is a perspective view of a step in the implantation of a discontinuous annuloplasty ring having enhanced visibility structure thereon; and
Figures 9A-9B are perspective views of two steps in the implantation of a C-shaped annuloplasty ring of the present invention utilizing various enhanced visibility structures on the annuloplasty ring, on an implantation tool, or at the implantation site.

### Description of the Preferred Embodiments

The present invention provides a number of structures, systems, and methods to enhance visibility of medical implants, especially during minimally-invasive cardiac surgeries. Of course, it will be understood that the embodiments disclosed herein may equally facilitate visibility of a particular implant in conventional open chest or other more invasive surgeries. Although the present invention is described and illustrated specifically with respect to heart valves and annuloplasty rings, the structures and methods for enhanced visibility can be utilized on other implants, including, but not limited to, vascular grafts, stents, etc.. In addition, the present invention discloses placement of an enhanced visibility structure on several embodiments of tools used in preparing a body cavity or delivering an implant. It should be understood that other such surgical site preparation and implant delivery tools could also be so adapted.

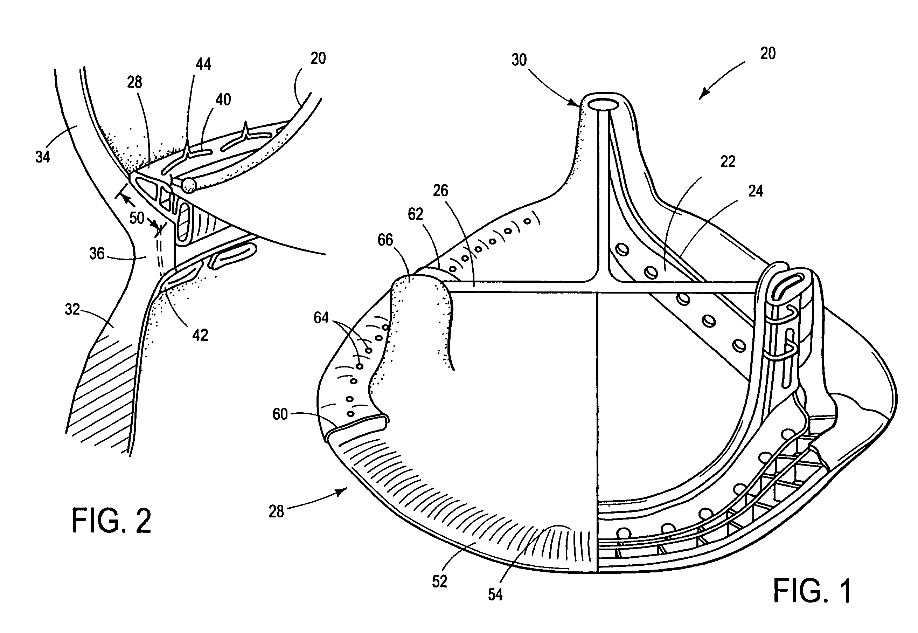

With reference to Figure 1, a tissue-type heart valve 20 typically includes a rigid wireform 22, a stent structure 24, a plurality of tissue leaflets 26, and a sewing ring 28 peripherally encircling an inflow end of the valve. The wireform 22 extends upward in three places and is cloth covered to define three upstanding commissures 30 between which the leaflets 26 are supported.

Figure 2 is a cross-section through a sidewall of an annulus region of a human heart depicting a ventricular wall 32 and an atrial wall 34 separated by an annulus 36. The annulus 36 extends inwardly to define a flow orifice between the ventricle and the atrium, and the heart valve 20 is seen attached to an upper side thereof, thus being located substantially within the atrium. The annulus shown corresponds to the aortic valve position between the left ventricle and left atrium. Of course, valves in the other positions can be adapted in accordance with present invention.

Structure for attaching the heart valve 20 to the annulus 36 is shown, comprising a plurality of sutures 40 passing through the annulus tissue and the sewing ring 28 and being supported on the ventricular side with pledgets 42. The pledgets 42 comprised small strips of flexible material (typically polymer) through which the sutures 40 pass and which help prevent the sutures from pulling through the annulus tissue when placed under tension. The sutures 40 typically extend downward through the sewing ring 28, annulus 36, through a pledget 42, and loop back up through the pledget, the annulus, and the sewing ring, with the two free ends knotted off as shown at 44. This closed-loop arrangement is repeated around the sewing ring 28 at closely spaced intervals.

Other suture arrangements, and other attachment configurations, are known for connecting the heart valve 20 to the annulus 36. For example, staples, barbs, adhesives, and other such attachment devices are either known or are contemplated. Therefore, the present invention should not be considered limited to suture-type attachment Instead, the sutures 40 will be more generally referred to as "attachment structure" so as to encompass various means of attachment.

In a basic form of the present invention, the attachment structure 40 (in this case sutures) is made of a material, or is treated, so as to have enhanced visibility. In this way, the particular placement of the attachment structure 40 can be more easily monitored by virtue of the enhanced contrast with the surrounding anatomical structures and portions of the implant that do not have enhanced visibility. For instance, the sewing ring 28 of the heart valve 20 extends radially for a dimension as indicated at 50 in Figure 2. The ring of sutures 40 is preferably radially positioned close to the middle of the sewing ring 28, or at approximately half of the dimension 50. By providing sutures 40 with enhanced visibility, this placement with respect to the sewing ring 28 is facilitated. Furthermore, locating the free ends of the sutures 40 to tie the knot 44 is easier because of their greater visibility. Finally, the integrity of the entire ring of sutures 40 at the end of the procedure can more easily be confirmed because of their enhanced visibility. An attendant advantage of utilizing sutures with enhanced visibility is the increased ability to spot stray bits of suture that have inadvertently been severed from the main lengths.

In the embodiment of Figure 2, the "attachment structure" also includes the pledgets 42. The surgical procedure of implanting the heart valve 20 can further be facilitated by providing pledgets 42 made of a material, or treated, so as to have enhanced visibility. Indeed, even in open-heart valve replacements, access to the ventricular side of the valve is limited. During a minimally-invasive surgery, not only is the access further restricted, but the available light from the operating room is dim or even non-existent. By providing pledgets 42 with enhanced visibility, the manipulation of a suture needle twice through the pledgets is made easier.

The present invention contemplates not only attachment structure having increased visibility, but also structure on the implant itself having increased visibility. For example, the sewing ring 28 shown in Figures 1 and 2 could be made of a material, or treated, so as to have enhanced visibility. In this regard, the entire sewing ring 28 may be rendered more visible, or only portions thereof. With reference specifically to Figure 1, the sewing ring 28 extends outward from the leaflet-supporting structure of the valve between an outer periphery 52 and an inner periphery 54. The entire external surface of the sewing ring 28 may be rendered more visible, or just one side or the other, either facing the annulus 36 or facing the atrium. Alternatively, circular markers may be provided following the outer periphery 52 and/or inner periphery 54 so as to define outer boundaries between which a suture needle or other attachment devices (e.g., staples) can be passed.

Not only can the enhanced visibility structure on the heart valve 20 itself provide a guide for the suturing process, but such structure may also be used prior to attachment during insertion of the valve. That is, enhanced visualization orientation markers may be provided on the sewing ring 28, or elsewhere on the valve 20. For example, commissure markers, such as indicated at 60, may extend radially outward on the sewing ring at the location of each of the commissures 30. Alternatively, cusp markers, such as indicated at 62, may be provided on sewing ring 28 at the circumferential midpoints between the commissures 30. In a more user-friendly approach, a circular series of suturing points 64 may be located around a sewing ring 28 as an aid in passing the sutures 40 through the midpoint of the sewing ring dimension 50. Moreover, the suturing points 64 may be optimally spaced to guide every pass of the suture needle. Still further, the uppermost tips of each of the commissures 30 may be capped with markers, such as is indicated at 66, having enhanced visibility to either help in the orientation of the valve, or provide beacons of a sort to help the surgeon avoid piercing the commissures 30, or leaflets 26 supported therebetween. In short, a variety of enhanced visibility structures can be incorporated into the tissue valve 20 to facilitate introduction and implantation in the heart.

Another technique is marking the anatomical tissue prior to implantation of the heart valve 20 (or other implant as the case may be). For example, sutures or other such markers may be attached to the annulus 36 at the preferred location of the commissures 30. Therefore, three such enhanced visibility sutures are first located around the annulus 36 to help the surgeon guide and orient the heart valve 20 into place. Alternatively, the innermost orifice of the sculpted annulus 36 may be indicated with enhanced visibility marking to facilitate centering of the valve 20. One possibility is to apply a substance having enhanced visibility to the annulus 36, which substance is biocompatible and will rapidly absorb or otherwise be integrated into the body. Moreover, the substance having enhanced visibility may also have therapeutic benefits. For example, a fibrin sealant incorporating an enhanced visibility component may be painted on the annulus 36, which not only helps guide the heart valve 20 into place, but helps in the healing process.

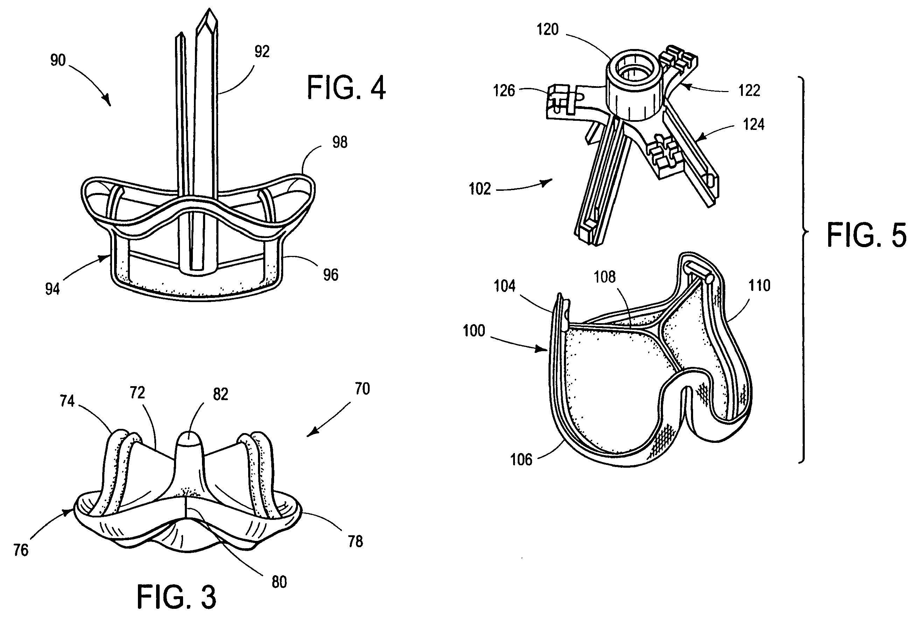

Figure 3 illustrates another embodiment of the tissue-type heart valve 70 having a plurality of leaflets 72 supported by upstanding commissures 74, and having a scalloped stent (not shown) and attached sewing ring 76. Again, various points or areas on the surface of the heart valve 70 may be provided with structure having enhanced visibility to facilitate insertion and implantation thereof. For example, an outer periphery 78 of the sewing ring 76 may include a marker line to help locate the peaks and valleys. Alternatively, markers, such as that shown at 80, may be provided at particular locations around sewing ring 76 for rotational orientation purposes. Again, as with the heart valve 20 in Figure 1, the tips 82 of the commissures may include high visibility caps or markers thereon.

Figure 4 illustrates an annulus sizer 90 having a shaft portion 92 and an obturator portion 94. The particular obturator 94 includes a cylindrical lower portion 96 and a scalloped upper portion 98. All or portions of the sizer 90 may be made of a material, or treated, so as to have enhanced visibility. That is, just the cylindrical lower portion 96 may be rendered more highly visible with the upper portion 98 being transparent to help visualize the fit between the lower portion and the heart annulus. Alternatively, markers can be provided on the peaks and/or valleys of the scalloped upper portion 98 for orientation purposes.

Still further, a substance having enhanced visibility may be coated on the sizer 90 to help size the annulus. The substance may be provided on the cylindrical portion 96, for example, so that the surgeon can insert and remove the sizer from the annulus and inspect the amount of substance that rubs off on the annulus. If the cylindrical portion 96 is smaller than the annulus, one would expect that only a segment, if any, of the periphery of the cylindrical portion would contact the annulus, and therefore only a small segment of enhanced visibility substance would rub off on the annulus. If the fit is good, however, one would expect that the entire annulus would be coated with a wide band of the substance. Consequently, the previously mentioned method of painting a circle of enhanced visibility substance on the annulus can be accomplished at the same time that the correct sizer 90 is determined.

Figure 5 illustrates, in exploded perspective, a highly flexible aortic heart valve 100 and an associated implantation holder 102. The heart valve 100 includes a peripheral stent having three upstanding commissures 104 separated by three arcuate cusps 106, the stent supporting a plurality of tissue leaflets 108 therebetween. The heart valve 100 further includes a sewing band 110 following the undulating line of the commissures 104 and cusps 106. The aortic heart valve 100 is designed to be attached not to the annulus using a conventional circular or scalloped sewing ring, but instead to the ascending aorta with sutures or other attachment means connecting the undulating sewing band 110 to the aorta.

It will be apparent to those of skill in the art that various of the previously described arrangements of enhancing the visibility of implants can be incorporated into the valve 100 for specific advantages. For instance, the outer peripheral edge of the sewing band 110 may be rendered more highly visible than the remainder of the valve 100 to indicate the outer boundary of the sewing region. Likewise, the inner boundary of the sewing band 110 may be indicated, or the entire sewing band may be rendered more highly visible. Alternatively, the commissure regions of the host aorta may be marked with structure or substance having enhanced visibility to facilitate orientation of the valve 100. Likewise, the commissures 104 of the valve 100 may be rendered more highly visible in conjunction with the anatomical marking to facilitate registration of the natural and prosthetic commissures.

Figure 5 also illustrates the holder 102 for the heart valve 100 comprising an upper handle attachment hub 120, three radially outwardly extending upper arms 122, and three downwardly angled lower legs 124. Each of the upper arms 122 extends into proximity with one of the commissures 104 of the valve 100 and is attached thereto with sutures, for instance. Likewise, each of the lower legs 124 extends into proximity with one of the cusps 106 and is attached thereto with sutures. Each of the upper arms 122 defines a generally radially extending channel 126 across which a suture attaching the respective cusp 104 traverses. The channel 126 receives a scalpel blade for severing the attachment suture and releasing that particular arm 122 from its cusp 104. In like manner, each of the lower legs 124 defines a scalpel channel for easily releasing the respective attachment suture from the valve 100.

To facilitate release of the valve 100 from the holder 102, the attachment structure, or portions of the holder 102, can be rendered highly visible. For instance, the floors or inner walls of the scalpel channels on the upper arms 122 and/or lower legs 124 can be painted or otherwise marked to help guide the surgeon during the task of severing the attachment sutures. Alternatively, the attachment sutures (or other attachment means, such as clips) may be made of a material, or treated, so as to have enhanced visibility. In this manner, the process of detaching the holder 102 is facilitated.

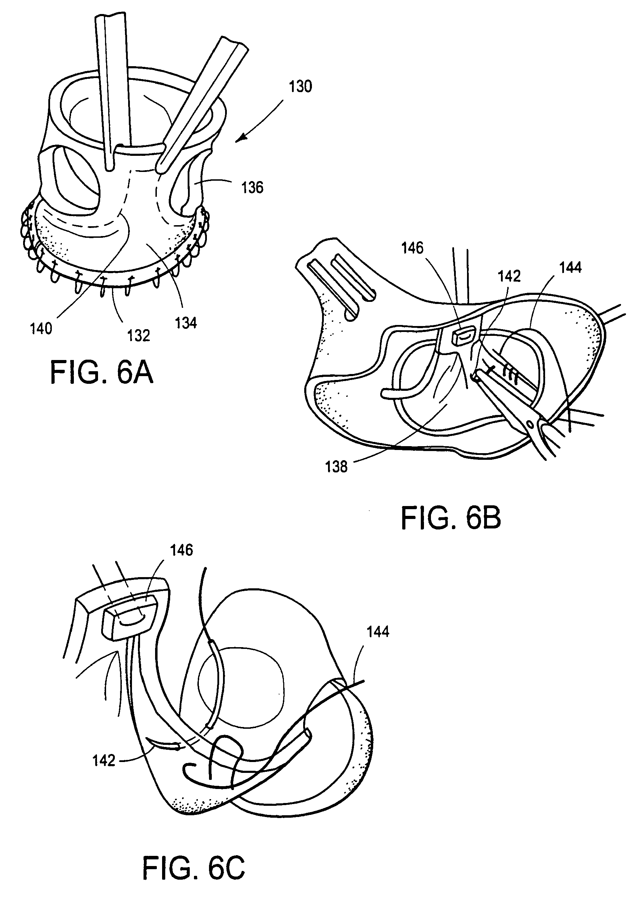

Figures 6A-6C illustrate several steps in trimming and implanting a stentless heart valve 130 utilizing the enhanced visualization structure of the present invention. The heart valve 130 includes an inflow rim 132, a tubular aortic wall 134, a pair of coronary artery orifices 136, and a plurality of tissue leaflets 138 supported within the aortic wall. This particular type of stentless valve 130 is typically fabricated from a segment of natural mammalian aorta (e.g., porcine) having an intact set of leaflets therein. The entire structure shown in Figure 6A may be implanted so that some of the natural aorta is either supplemented or replaced. Alternatively, a portion of the aortic wall 134 may be trimmed, as indicated, to provide a less than total aortic replacement. To facilitate the trimming procedure, a line of sutures or other markers 140 having enhanced visibility is provided on the exterior of the aortic wall 134. Moreover, because the aortic wall 134 is trimmed so that the marker line 140 remains on the prosthetic valve, the insertion step is facilitated.

Figures 6B and 6C illustrate the implantation process wherein a curved suture needle 142 having a following suture thread 144 is repeatedly passed through the natural tissue and through the remaining aortic wall 134 along the marker line 140. Pledgets 146 may be utilized at the commissure regions of the prosthesis 130. Again, the suture thread 144 and/or pledgets 146 may be made of a material, or treated, so as to have enhanced visibility to facilitate the implantation procedure. Also, the marker line 140 may be provided both on the exterior and the interior of the aortic wall 134.

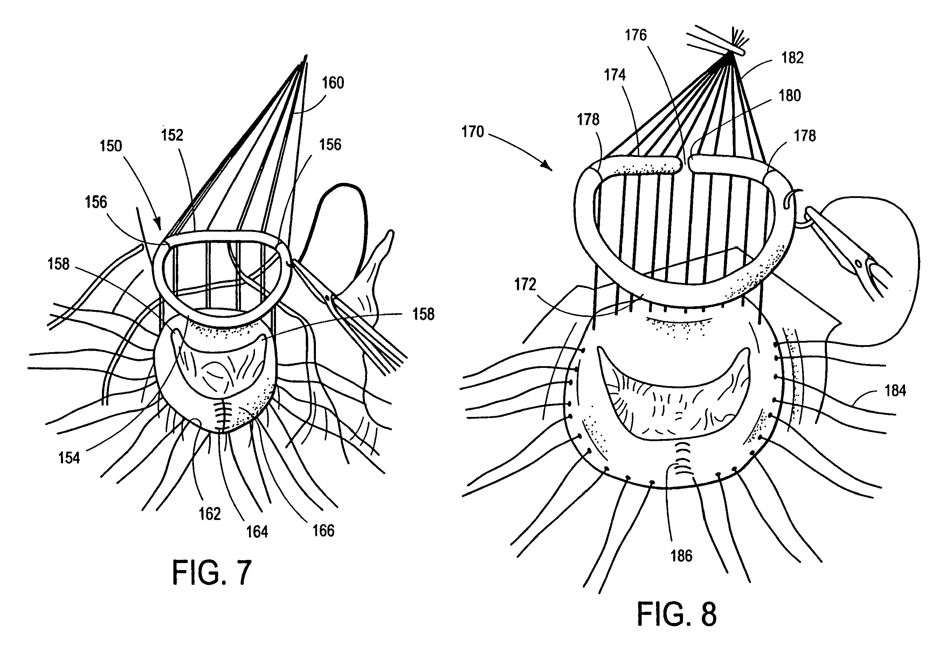

Figure 7 illustrates a step in the implantation of an annuloplasty ring 150 with the help of the enhanced visualization structure of the present invention. The annuloplasty ring 150 illustrated has a continuous D-shaped profile with a substantially straight anterior side 152 and a curvilinear posterior side 154. The extent of the anterior side 152 is delimited by a pair of commissure markers 156 that are preferably made of a material, or treated, so as to have enhanced visibility. In the mitral position, the heart valve comprises a bi-leaflet structure having a pair of commissures 158. The commissure markers 156 therefore facilitate proper registration of the annuloplasty ring 150 with the host commissures 158. Alternatively, the entire ring 150 may be made of a material, or coated, so as to have enhanced visibility.

The annuloplasty ring 150 is seen being implanted in position along a plurality of attachment sutures 160. As is conventional, the sutures are first anchored around the heart valve annulus 162 at predetermined intervals and passed through the suture-permeable material of the ring 150 outside the body. The ring 150 is then simply advanced into place, and each pair of sutures 160 tied off. In one embodiment of present invention, the sutures 160 are made of a material, or are treated, so as to have enhanced visibility. In this way, proper placement and spacing of the sutures 160 is facilitated.

Figure 7 also illustrates a row of sutures 164 used to constrict the host valve by securing previously severed portions of a posterior leaflet 166 together. In accordance with present invention, these "cinching" sutures 164 are made of a material, or are treated, so as to have enhanced visibility. Because of the enhanced visibility of the sutures 164, the surgeon can more easily insure the quality of the suture line joining the portions of the posterior leaflet 166.

Figure 8 illustrates another annuloplasty ring 170 having a curvilinear posterior side 172 and a generally straight anterior side 174 formed by two segments separated at a discontinuity 176. Again, enhanced visibility commissure markers 178 may be provided. In addition, the free ends of the segments defining the anterior leaflet 174 may including a cap or coating 180 of a material having enhanced visibility. In this way, the discontinuity 176 can be more easily centered along the anterior side of the host annulus.

Figure 8 also illustrates a step of suturing the annuloplasty ring 170 into place, wherein a first plurality of sutures 182 has previously been passed through the anterior portion of the annulus and then through the anterior side 174. A second plurality of sutures 184 are secured around the posterior portion of the annulus, with the surgeon in the process of passing each suture through the posterior side 172 of the ring 170. In addition, the posterior leaflet has been repaired with sutures 186. Either or both of the sutures 184,186 may be made of a material, or treated, so as to have enhanced visibility.

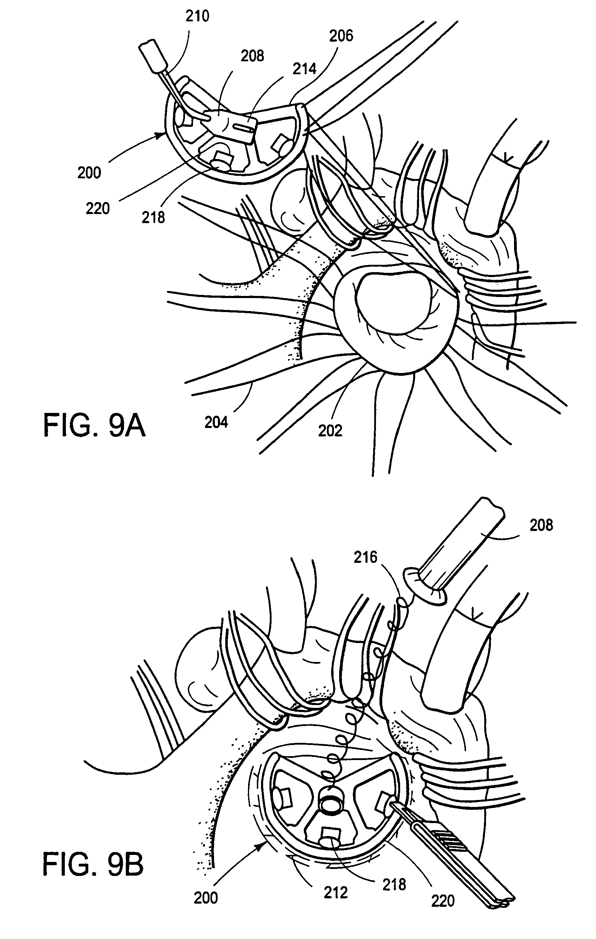

Figures 9A and 9B illustrate two steps in implantation of an annuloplasty ring 200 that has a substantially C-shape and is designed to be secured around a posterior side of the annulus. Figure 9A illustrates a distended posterior side 202 that is corrected in Figure 9B to the shape of the annuloplasty ring 200. Again, a plurality of sutures 204 are first anchored around the host annulus and then threaded through the annuloplasty ring 200 so as to enable advancement of the ring into place. The sutures 204 may be made of a material, or treated, so as to have enhanced visibility in accordance with the present invention.

The annuloplasty ring 200 is manipulated into place within the annulus using a template 206 having a lanyard 208 and handle 210 attached thereto. The annuloplasty ring 200 is removably secured around an arcuate periphery of the template 206, and is advanced into place and the sutures 204 tied off, as indicated at 212 in Figure 9B, while still on the template. To help the surgeon formed the knots 212, the lanyard 208 is attached to the template using a plurality of sutures 214. The lanyard 208 includes a guide structure to enable the surgeon to easily sever the sutures 214. In accordance with the present invention, the attachment sutures 214 are made of a material, or are treated, so as to have enhanced visibility and facilitate detachment of the lanyard 208 from the template 206.

After detachment of the lanyard 208, it may still be coupled to the template 206 via a tether 216. After the knots 212 are secured, the template 206 is detached from the annuloplasty ring 200 and the template removed via the tether 216. To accomplish this, the template 206 is removably secured to the ring 200 using a plurality of attachment sutures 218. Each suture 218 is typically secured at both ends to the template 206, threaded through the annuloplasty ring 200 at one or more locations, and passed over a cutting guide 220. By severing each suture 218 at the cutting guide 220, the template 206 can be separated from the annuloplasty ring 200, with the free ends of the sutures 218 being pulled free from the ring. Again, to facilitate this separation, the sutures 218 and/or cutting guide 220, are made of a material, or are treated, so as to have enhanced visibility.

### Definition of Enhanced Visibility

In the context of the present invention, "enhanced visibility" in general means the optical characteristic of a structure that enables it to be more easily seen in environments where incident light is limited, or enables it to be seen where there is no incident light. "Seen" refers to being visualized with the naked eye, or the naked eye through a scope such as an endoscope, and is distinguished from being visualized by an X-ray or other such imaging device. There are a number of different means for achieving "enhanced visibility," some of which can be used in conjunction with others, and some of which have particular advantages over the others depending on the situation. In the present invention, the means of providing "enhanced visibility" is to utilize a luminescent structure having the property that incident or external light atomically excites the structure so as to emit light for a period of time. In other words, the structure glows even after the incident light is removed. Such a structure can therefore be utilized in darker environments, such as difficult to reach minimally-invasive surgical sites. It should therefore be understood that the following categories of "enhanced visibility" may all be used in conjunction with apparatus as presently claimed, but that each category has unique advantages and thus should not be necessarily be lumped together in terms of patentability of the entire group. in the context of minimally-invasive surgery. Metallic sutures can be used in any of the embodiments disclosed above so as to reflect incident light and be more easily seen. Gold in particular is highly reflective and thus particularly suited to enhance the visibility of an implant, or to facilitate the implantation procedure. However, gold is relatively expensive and thus stainless-steel or platinum, or other suitably reflective metal, provide more economically feasible alternatives.

In another specific embodiment, the structure that is highly reflective may be incorporated into a medical implant. For instance, any of the markers associated with the implants disclosed above may be made highly reflective. Alternatively, the entire sewing ring of any of the heart valves disclosed above, or the entire surface of the annuloplasty rings disclosed, may be made highly reflective by incorporation of biocompatible particles having high reflectivity. The end result is a structure that shines or sparkles when exposed to an external light source. Even in the dark cavities found in minimally-invasive surgeries, wherein the external light source may be limited to that provided at the end of a catheter, the reflective structure can be easily located.

Metallic coatings on the fabric of medical implants may be provided to reflect fiber-optic light and enhance visualization. The coatings can be applied by a number of conventional techniques, including thermal vaporization in a vacuum, sputtering (magnetron or ion beam sputtering) in a vacuum, or chemical vapor deposition. Moreover, specific surfaces of the implants may be coated using a mask so that the subsequently formed markers have a particular shape or orientation. For instance, the circumferential suture line 64 shown in Figure 1, or the peripheral sewing ring line 78 shown in Figure 3 may be formed by a masking process.

Another type of highly reflective structure that can be incorporated into a medical implant is one which is highly polished, whether it be metallic or otherwise. For example, certain ceramics such as pyrolytic carbon or sapphire may be utilized if polished to shine. Such ceramic coatings are typically vapor deposited in a vacuum, for example. Such materials can be polished to be highly reflective if they are deposited on a hard surface, as opposed to a fabric.

Still another variation on the highly reflective structure is a multi-faceted surface, such as in vehicle reflectors or glitter. A multi-faceted surface includes a

### Luminescence, Fluorescence, Phosphorescence, Chemiluminescence

According to the Encyclopedia Britannica, "luminescence" pertains to all light phenomena not caused solely by a rise of temperature. The terms phosphorescence and fluorescence are generally grouped under luminescence, but the distinction between them remains open to discussion. With respect to organic molecules, the term phosphorescence means light emission caused by electronic transitions between levels of different multiplicity, whereas the term fluorescence is used for light emission connected with electronic transitions between levels of like multiplicity. The situation is different in the case of inorganic phosphors. Phosphorescence was first used to describe the persistent luminescence (afterglow) of phosphors. Fluorescence, on the other hand, is an almost instantaneous effect, ending almost immediately after the source of excitation. In terms of the present invention, therefore, phosphorescence will be used to define structure that emits light during exposure to and after removal of an external light source (i.e., it glows), while fluorescence will be used to define structure that emits light during exposure to an external light source.

Luminescent materials or coatings can be utilized for any of the structures described above having "enhanced visibility." That is, any of the markers, sutures, implant structures, tools, or other structure useful during the installation of an implant may be made either phosphorescence or fluorescent to facilitate the installation procedure.

In a specific example, the entire sewing ring of any of the heart valves disclosed above, or the entire annuloplasty rings disclosed, may be made fluorescent so as to emit light during a minimally invasive procedure so long as an external source, such as at the end of a catheter, is provided. Alternatively, any of the various sutures disclosed above, either for securing the prosthesis to the host tissue or for securing an installation tool to the prosthesis, may be made fluorescent. Moreover, these same structures may be made phosphorescent so that they glow even when an external source is absent, or is occluded or otherwise dimmed.

In a further alternative, structure that emits light, or glows, upon excitation of a particular wavelength of light may be used. For example, a so-called "black light" can be used at the surgery site to more effectively illuminate the luminescent structure.

A luminescent substance or compound may be particularly useful in marking certain anatomical landmarks to assist in the implantation procedure. So long as the luminescent substance is biocompatible, it may be painted or otherwise deposited onto a particular structure, for example a heart valve annulus, to help the surgeon in properly aligning and orienting the implant.

Chemiluminescence pertains to a structure that emits light at room temperature from a chemical reaction. The present invention also contemplates structure in the implant that is chemiluminescent, so that the absence or limitation of an external light source is even less significant for continued visibility of the implant.

While the foregoing is a complete description of the preferred embodiments of the invention, various alternatives, modifications, and equivalents may be used. It will be obvious that certain other modifications may be practiced within the scope of the appended claims.

## Claims

1. An enhanced visibility medical implant comprising:
a medical implant (20);
**characterized in that**
luminescent structure (28) is provided on the medical implant to facilitate location of the structure in low light environments.

2. An implant as claimed in Claim 1, wherein the luminescent structure is fluorescent.

3. An implant as claimed in Claim 1, wherein the luminescent structure is phosphorescent.

4. An implant as claimed in Claim 1, wherein the luminescent structure is chemiluminescent.

5. An implant as claimed in any preceding claim, wherein the medical implant comprises a cardiovascular implant.

6. An implant as claimed in any preceding claim, further comprising:
luminescent attachment structure (40).

7. An implant as claimed in Claim 6, wherein the luminescent attachment structure is selected from the group consisting of:
fluorescent;
phosphorescent; and
chemiluminescent.

8. An implant as claimed in any one of Claims 6 to 7, wherein the luminescent attachment structure comprises sutures (40).

9. An implant as claimed in any one of Claims 6 to 8, wherein the luminescent attachment structure is selected from the group consisting of:
staples;
barbs; and
adhesive.

10. An implant as claimed in any one of Claims 6 to 9, wherein the luminescent attachment structure is configured to register with the luminescent structure on the medical implant.

11. An implant as claimed in Claim 10, wherein the medical implant includes a suture-permeable portion and the luminescent structure comprises suture locator markings (64) on the suture-permeable portion, and wherein the luminescent attachment structure comprises sutures (40).

12. An implant as claimed in any preceding claim, wherein the medical implant has a fabric portion, and wherein a luminescent marking is provided on the fabric portion.

13. An implant as claimed in Claim 12, wherein the medical implant is a heart valve (20, 70), and the fabric portion is a sewing ring (28, 76).

14. An implant as claimed in Claim 13, wherein the luminescent marking comprises at least one rotational orientation marking (80) on the sewing ring (76).

15. An implant as claimed in Claim 13, wherein the luminescent marking comprises a circumferential line de-limiting an inner or outer boundary of the sewing ring.

16. An implant as claimed in Claim 12, wherein the medical implant is a heart valve and the heart valve includes upstanding commissures (30) having fabric coverings, and wherein the luminescent marking comprises a marker on the fabric of at least one of the commissures.

17. An implant as claimed Claim 12, wherein the medical implant is an annuloplasty ring (150).

18. An implant as claimed in Claim 17, wherein the luminescent marking comprises at least one rotational orientation marker on the annuloplasty ring.

19. An implant as claimed in Claim 17, wherein the annuloplasty ring is discontinuous having two free ends, and wherein the luminescent marking comprises a marker on at least one of the free ends.

## Patentansprüche

1. Eine verbesserte Sichtbarkeit aufweisendes medizinisches Implantat mit einem medizinisches Implantat (20), **dadurch gekennzeichnet, dass** auf dem medizinischen Implantat eine lumineszierende Struktur (28) vorhanden ist, um das Auffinden der Struktur unter schlechten Lichtverhältnissen zu erleichtern.

2. Implantat gemäß Anspruch 1, wobei die lumineszierende Struktur fluoreszierend ausgebildet ist.

3. Implantat gemäß Anspruch 1, wobei die lumineszierende Struktur phosphoreszierend ausgebildet ist.

4. Implantat gemäß Anspruch 1, wobei die lumineszierende Struktur chemilumineszend ausgebildet ist.

5. Implantat gemäß einem der vorangehenden Ansprüche, wobei das Implantat ein kardiovaskuläres Implantat umfasst.

6. Implantat gemäß einem der vorangehenden Ansprüche, wobei weiterhin eine lumineszierende Anfügestruktur (40) vorhanden.

7. Implantat gemäß Anspruch 6, wobei die lumineszierende Anfügestruktur aus einer Gruppe gewählt ist, die besteht aus:
fluoreszierend,
phosphoreszierend und
chemilumiszend.

8. Implantat gemäß Anspruch 6 oder 7, wobei die lumineszierende Anfügestruktur Nähte (40) umfasst.

9. Implantat gemäß einem der Ansprüche 6 bis 8, wobei die lumineszierende Anfügestruktur aus einer Gruppe gewählt ist, die aus
Klammern,
Haken und
Haftmittel
beteht.

10. Implantat gemäß einem der Ansprüche 6 bis 9, wobei die lumineszierende Anfügestruktur derart ausgestaltet ist, dass sie mit der lumineszierenden Struktur auf dem Implantat zusammenpasst.

11. Implantat gemäß Anspruch 10, wobei das medizinische Implantat einen nahtdurchlässigen Abschnitt aufweist, wobei die lumineszierende Struktur Nahtpositionsmarkierungen (64) an dem nahtdurchlässigen Abschnitt umfasst und wobei die lumineszierende Anfügestruktur Nähte (40) umfasst.

12. Implantat gemäß einem der vorangehenden Ansprüche, wobei das medizinische Implantant einen Gewebeabschnitt aufweist und wobei auf dem Gewebeabschnitt eine lumineszierende Markierung vorhanden ist.

13. Implantat gemäß Anspruch 12, wobei das medizinische Implantat eine Herzklappe (20, 70) und der Gewebeabschnitt ein Nahtring (28, 76) ist.

14. Implantat gemäß Anspruch 13, wobei die lumineszierende Markierung mindestens eine Drehrichtungsmarkierung (80) auf dem Nahtring (76) umfasst.

15. Implantat gemäß Anspruch 13, wobei die lumineszierende Markierung eine den äußeren und den inneren Umfang des Nahtrings begrenzende umlaufende Linie umfasst.

16. Implantat gemäß Anspruch 12, wobei das medizinische Implantat eine Herzklappe ist und wobei die Herzklappe aufrecht stehende Kommissuren (30) aufweist, welche über Gewebeabdeckungen verfügen, und wobei die lumineszierende Markierung einen Marker auf dem Gewebe wenigstens einer der Kommissuren umfassen.

17. Implantat gemäß Anspruch 12, wobei das medizinische Implantat ein Annuloplastikring (150) ist.

18. Implantat gemäß Anspruch 17, wobei die lumineszierende Markierung mindestens einen Drehrichtungsmarker auf dem Annuloplastikring umfasst.

19. Implantat gemäß Anspruch 18, wobei der Annuloplastikring unterbrochen ist sowie zwei freie Enden aufweist und wobei die lumineszierende Markierung mindestens einen Marker an wenigstens einem der freien Enden umfasst.

## Revendications

1. Implant médical à visibilité améliorée comprenant :
un implant médical (20) ;
**caractérisé en ce que**
une structure luminescente (28) est prévue sur l'implant médical pour faciliter la localisation de la structure dans les environnements de faible luminosité.

2. Implant selon la revendication 1, dans lequel la structure luminescente est fluorescente.

3. Implant selon la revendication 1, dans lequel la structure luminescente est phosphorescente.

4. Implant selon la revendication 1, dans lequel la structure luminescente et chimioluminescante.

5. Implant selon l'une quelconque des revendications précédentes, dans lequel l'implant médical comprend un implant cardiovasculaire.

6. Implant selon l'une quelconque des revendications précédentes, comprenant en outre :
une structure de fixation luminescent (40).

7. Implant selon la revendication 6, dans lequel la structure de fixation luminescente est sélectionné parmi le groupe consistant en :
fluorescent ;
phosphorescent ; et
chimioluminescent.

8. Implant selon l'une quelconque des revendications 6 à 7, dans lequel la structure de fixation luminescente comprend des sutures (40).

9. Implant selon l'une quelconque des revendications 6 à 8, dans lequel la structure de fixation luminescente est sélectionnée parmi le groupe consistant en :
agrafes;
fils ; et
adhésifs.

10. Implant selon l'une quelconque des revendications 6 à 9, dans lequel la structure de fixation luminescente est configurée pour coïncider avec la structure luminescente sur l'implant médical.

11. Implant selon la revendication 10, dans lequel l'implant médical inclut une partie perméable à la suture et la structure luminescente comprend des marquages (64) localisateurs de sutures sur la partie perméable à la suture, et dans lequel la structure de fixation luminescente comprend des sutures (40).

12. Implant selon l'une quelconque des revendications précédentes, dans lequel l'implant médical comporte une partie de tissu, et dans lequel un marquage luminescent est prévu sur la partie de tissu.

13. Implant selon la revendication 12, dans lequel l'implant médical est une valve cardiaque (20, 70) et la partie de tissu est un anneau de couture (28, 76).

14. Implant selon la revendication 13, dans lequel le marquage luminescent comprend au moins un marquage (80) à orientation rotationnelle sur l'anneau de couture (76).

15. Implant selon la revendication 13, dans lequel le marquage luminescent comprend une ligne circonférentielle délimitant une bordure interne ou externe de l'anneau de couture.

16. Implant selon la revendication 12, dans lequel l'implant médical est une valve cardiaque et la valve cardiaque inclut des commissures droites (30) comportant des revêtements de tissu, et dans lequel le marquage luminescent comprend un marqueur sur le tissu d'au moins une des commissures.

17. Implant selon la revendication 12, dans lequel l'implant médical est un anneau d'annuloplastie (150).

18. Implant selon la revendication 17, dans lequel le marquage luminescent comprend au moins un marqueur à orientation rotationnelle sur l'anneau d'annuloplastie.

19. Implant selon la revendication 17, dans lequel l'anneau d'annuloplastie est discontinu comportant deux extrémités libres, et dans lequel le marquage luminescent comprend un marqueur sur au moins une des extrémités libres.
